# EUROPÄISCHE PATENTSCHRIFT

(11) **EP 1 929 001 B1**
(45) Veröffentlichungstag und Bekanntmachung des Hinweises auf die Patenterteilung: **28.04.2010**
(21) Anmeldenummer: 06806768.5
(22) Anmeldetag: 14.09.2006
(51) Int. Cl.: C12N 9/04

(54) **NEUE DEHYDROGENASEN, DEREN DERIVATE UND EIN VERFAHREN ZUR HERSTELLUNG VON OPTISCH AKTIVEN ALKANOLEN**
NOVEL DEHYDROGENASES, THE DERIVATIVES THEREOF, AND METHOD FOR THE PRODUCTION OF OPTICALLY ACTIVE ALKANOLS
NOUVELLES DESHYDROGENASES, LEURS DERIVES, ET UN PROCEDE POUR PRODUIRE DES ALCANOLS OPTIQUEMENT ACTIFS

(30) Priorität: 19.09.2005 DE 102005044736
(43) Veröffentlichungstag der Anmeldung: 11.06.2008
(73) Patentinhaber: BASF SE, 67056 Ludwigshafen (DE)
(72) Erfinder: BREUER, Michael, 64285 Darmstadt (DE); FRIEDRICH, Thomas, 64283 Darmstadt (DE); KESSELER, Maria, 68239 Mannheim (DE)
(86) Internationale Anmeldenummer: PCT/EP2006/066336
(87) Internationale Veröffentlichungsnummer: WO 2007/033928

(56) Entgegenhaltungen:
- EP-A1- 1 152 054
- WO-A2-2004/090094
- WO-A2-2005/033094
- WO-A2-2005/108590
- PANKAJ SONI ET AL: "Biotransformations for the production of the chiral drug (S)-Duloxetine catalyzed by a novel isolate of Candida tropicalis" APPLIED MICROBIOLOGY AND BIOTECHNOLOGY, SPRINGER-VERLAG, BE, Bd. 67, Nr. 6, 1. Juni 2005 (2005-06-01), Seiten 771-777, XP019331870 ISSN: 1432-0614
- LIU H ET AL: "CHEMO-ENZYMATIC SYNTHESIS OF THE ANTIDEPRESSANT DULOXETINE AND ITS ENANTIOMER" CHIRALITY, WILEY-LISS, NEW YORK, US, Bd. 12, Nr. 1, 2000, Seiten 26-29, XP009000316 ISSN: 0899-0042

## Beschreibung

Die vorliegende Erfindung betrifft Proteine, die eine enzymatische Aktivität zur Reduktion von substituierten Alkanonen aufweisen. Die Erfindung betrifft weiterhin Nukleinsäuren, die für diese Proteine kodieren, Nukleinsäurekonstrukte, Vektoren, genetisch veränderte Mikroorganismen sowie Verfahren zur Herstellung von optisch aktiven substituierten Alkanolen, insbesondere (S)-Alkanolen wie z.B. (S)-3-Methylaminor1-(2-thienyl)-(S)-propanol.

### Stand der Technik:

Dehydrogenasen sind vielseitig einsetzbare Katalysatoren zur enantioselektiven Reduktion von Aldehyden oder Ketonen zu den korrespondierenden Alkoholen. Man unterscheidet dabei (R)- und (S)-spezifische Dehydrogenasen. Diese Katalysatoren werden im verstärkten Maß für die industrielle Synthese von optisch aktiven Alkoholen eingesetzt. Optische Aktivität ist die Voraussetzung für die selektive Wirkung von vielen Pharma- und Agrowirkstoffen. Hier kann das eine Enantiomer die gewünschte, das andere Enantiomer eine genotoxische Wirkung haben. Aus diesem Grund werden in der Synthese von Pharma- und Agrowirkstoffen zur Herstellung von optisch aktiven Alkoholen Katalysatoren eingesetzt, die die notwendige Stereospezifität besitzen.

In der Literatur (vgl. EP-A-0 273 658) sind Synthesewege zum Duloxetinealkohol und Duloxetin beschrieben. Nachteil dieser Synthesewege ist, dass die Synthese zum racemischen Alkoholgemisch führt und anschließend eine Racematspaltung durch Überführung des Racemats in eine Mischung aus Diastereomeren durch Salzbildung mit einem optisch aktiven Gegenion erforderlich ist. Anschließend erfolgt eine physikalischen Trennung der Diastereomere. Hieraus resultieren hohe Verfahrenskosten aufgrund wiederholter Fest-Flüssigtrennungen und der erhöhte Einsatzstoffaufwand durch Zugabe eines optisch aktiven Salzes zur Trennung.

Einen preisgünstigeren Zugang zum Duloxetinealkohol würde die stereospezifische Reduktion des 3-Methylamino-1-(2-thienyl)-propanon bieten.

EP1152054 beschreibt neue Carbonylreduktasen und deren Verwendung zur Herstellung von tert. Butyl (3R,5S)- 6-chloro-3,5 dihydroxyhexanoat.

WO04/90094 beschreibt Camithin-Dehydrogenasen und deren Verwendung zur Herstellung von optisch aktiven Alkanolen.

### Kurze Beschreibung der Erfindung:

Der Erfindung lag daher die Aufgabe zugrunde, einen Weg zur stereospezifischen Reduktion von substituierten Alkanonen zu finden.

Diese Aufgabe wurde gelöst durch die Bereitstellung von neuen Dehydrogenasen, die zur stereospezifischen Katalyse der obigen Reaktion befähigt sind.

Ein erster Gegenstand der Erfindung betrifft ein Verfahren zur mikrobiologischen, insbesondere enantioselektiven Herstellung von substituierten Alkanolen der Formel I 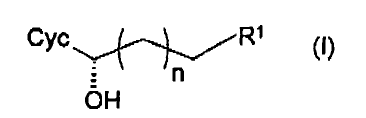 worin
- n: für einen ganzzahligen Wert von 0 bis 5, insbesondere 0, 1 oder 2, steht;
- Cyc: für einen gegebenenfalls substituierten, ein- oder mehrkernigen, gesättigten oder ungesättigten, carbocyclischen Ring, insbesondere einen gegebenen- falls substituierten, ungesättigten, einkemigen heterocyclischen Ring, steht, und
- R¹: für Halogen, SH, OH, NO₂, NR²R³ oder NR²R³R⁴⁺X⁻, insbesondere Halogen oder NR²R³ steht, wobei R², R³ und R⁴ unabhängig voneinander für H oder einen Niedrigalkyl- oder Niedrigalkoxy-Rest stehen und X⁻ für ein Gegenion steht,
wobei man in einem ein Alkanon der Formel II 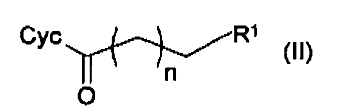 worin n, Cyc und R¹ die oben angegebenen Bedeutungen besitzen, enthaltenden Medium,
a) einen eine Dehydrogenase mit der Polypeptidsequenz SEQ ID NO:2 oder NO:4, oder mit einer Polypeptidsequenz, bei der bis zu 25% der Aminosäurereste gegenüber SEQ ID NO: 2 oder NO:4 durch Deletion, Insertion, Substitution oder einer Kombination davon verändert sind, produzierenden Mikroorganismus kultiviert, oder
b) eine Dehydrogenase wie unter a) genannt inkubiert,
wobei die Verbindung der Formel II zur Verbindung der Formel I enzymatisch reduziert wird, und man das in im Wesentlichen enantiomerenreiner Form gebildete Produkt isoliert.

Vorzugsweise verwendet man bei diesen Verfahren ein Enzym mit einer Polypeptidsequenz gemäß SEQ ID NO:2 oder NO:4.

Derartige Enzyme können beispielsweise aus Mikroorganismen der Gattung Candida isoliert werden.

In einer besonders bevorzugten Ausführungsform des Verfahrens ist das Enzym ausgewählt unter Enzymen, welche eine Aminosäuresequenz gemäß SEQ ID NO: 2, 4, umfassen, oder von davon abgeleiteten Nukleinsäuresequenzen kodiert werden; und bei der bis zu 25% der Aminosäurereste durch Deletion; Insertion, Substitution oder einer Kombination davon verändert sind, welche Dehydrogenase-Aktivität besitzen und die enantioselektive Synthese einer Verbindung der Formel I katalysieren.

Beispielsweise kann das Enzym mit Dehydrogenase-Aktivität von einem Nukleinsäuresequenz gemäß SEQ ID NO:9 oder NO:3 oder Enzymen bei denen 25% der Aminosäurereste durch Deletion; Insertion, Substitution oder einer Kombination davon verändert sind.

Vorzugsweise wird das erfindungsgemäße Verfahren unter Zugabe von Reduktionsäquivalenten (NADH oder NADPH) oder die bei der Umsetzung verbrauchten Reduktionsäquivalente regenerierenden (biochemischen oder elektrochemischen) Bedingungen durchgeführt.

Weiterhin ist es bevorzugt, die Umsetzung der Verbindung der allgemeinen Formel II in Gegenwart eines Mikroorganismus erfolgen zu lassen, der ausgewählt ist unter Bakterien der Familien Enterobacteriaceae, Pseudomonadaceae, Rhizobiaceae, Streptomycetaceae und Nocardiaceae. Der Mikroorganismus kann insbesondere ein rekombinanter Mikroorganismus sein, der mit einem Nukleinsäurekonstrukt transformiert ist, welche für ein erfindungsgemäßes Enzym mit Dehydrogenase-Aktivität gemäß obiger Definition kodiert.

Gegenstand der Erfindung ist insbesondere ein Verfahren gemäß obiger Definition, wobei man
a) einen ein Enzym mit Dehydrogenase-Aktivität produzierenden Mikroorganismus aus einer natürlichen Quelle isoliert oder rekombinant herstellt,
b) diesen Mikroorganismus vermehrt,
c) aus dem Mikroorganismus das Enzym mit Dehydrogenase-Aktivität gegebenenfalls isoliert oder eine dieses Enzym enthaltende Proteinfraktion herstellt, und
d) den Mikroorganismus gemäß Stufe b) oder das Enzym gemäß Stufe c) in ein Medium überfährt, das eine Verbindung der Formel I enthält.

Die Erfindung betrifft weiterhin ein Verfahren zur Herstellung einer Verbindung der allgemeinen Formel III 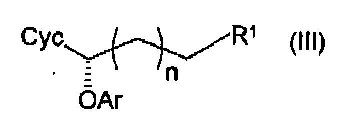 worin n, Cyc, und R¹ die oben angegebenen Bedeutungen besitzen und Ar für einen ein- oder mehrkernigen, gegebenenfalls substituierten Aryl-Rest steht, und
wobei man
a) zunächst auf mikrobiologischem Weg gemäß der Definition in einem der vorhergehenden Ansprüche eine Verbindung der Formel I erstellt; und
b) die Verbindung der Formel I mit eine aromatischen Verbindung der Formel IV

   Ar-Y (IV)

   worin Ar die oben angegebenen Bedeutungen besitzt und Y für eine Abgangsgruppe steht, umsetzt und
c) die Verbindung der Formel III isoliert und gegebenenfalls in ein pharmazeutisch verträgliches Säureadditionssalz, wie z.B. Oxalate überführt.

Vorzugsweise stellt man dabei eine Verbindung der Formel III her, worin Ar für 1-Naphthyl, Cyc für 2-Thienyl, R¹ für Monomethylamino und n für 1 steht.

Ein weiterer Gegenstand der Erfindung betrifft Polypeptide, welche eine Aminosäuresequenz gemäß SEQ ID NO: 2 oder 4 umfassen, oder von davon abgeleiteten Nukleinsäuresequenzen kodiert werden; und bei der bis zu 25% der Aminosäurereste durch Deletion; Insertion, Substitution oder einer Kombination davon verändert sind, welche Dehydrogenase-Aktivität besitzen und die enantioselektive Synthese einer Verbindung der Formel I katalysieren.

Gegenstand der Erfindung sind außerdem kodierende Nukleinsäuresequenzen, umfassend die kodierende Sequenz für ein Polypeptid gemäß obiger Definition.

Weiterhin betrifft die Erfindung Expressionskassetten, umfassend in operativer Verknüpfung mit wenigstens einer regulativen Nukleinsäuresequenz eine kodierende Nukleinsäuresequenz gemäß obiger Definition.

Ein weiterer Gegenstand der Erfindung sind rekombinante Vektoren, umfassend wenigstens eine solche Expressionskassette.

Die Erfindung betrifft auch prokaryotische oder eukaryotische Wirte, welche mit wenigstens einem erfindungsgemäßen Vektor transformiert sind.

### Detaillierte Beschreibung der Erfindung:

### A. Allgemeine Begriffe und Definitionen

Werden keine anderen Angaben gemacht, so gelten folgende allgemeine Bedeutungen:
"Halogen" steht für Fluor, Chlor, Brom oder Jod, insbesondere Fluor oder Chlor.
"Niedrigalkyl" steht für geradkettige oder verzweigte Alkylreste 1 bis 6 C-Atomen, wie Methyl, Ethyl, i- oder n-Propyl, n-, i-, sec.- oder tert.-Butyl, n-Pentyl oder 2-MethylButyl, n-Hexyl, 2-Methyl-pentyl, 3-Methyl-pently, 2-Ethyl-butyl.
"Niedrigalkenyl" steht für die ein- oder mehrfach, vorzugsweise einfach oder zweifach ungesättigten Analoga oben genannter Alkylreste mit 2 bis 6 Kohlenstoffatomen, wobei die Doppelbindung in beliebiger Position der Kohlenstoffkette liegen kann.
"Niedrigalkoxy" steht für die Sauerstoff-terminierten Analoga obiger Alkylreste.
"Aryl" steht für einen ein- oder mehrkernigen, vorzugsweise ein- oder zweikernigen, gegebenenfalls substituierten aromatischen Rest, insbesondere für Phenyl oder für ein über eine beliebige Ringposition gebundenes Naphthyl, wie 1- oder 2-Naphthyl. Diese Arylreste können gegebenenfalls 1 oder 2 gleiche oder verschiedene Substituenten tragen, ausgewählt unter Halogen, Niedrigalkyl, Niedrigalkoxy gemäß obiger Definition oder Trifluormethyl.

### B. Substituierte Alkanone, (S)-Alkanole und Derivate davon

Erfindungsgemäß durch enzymatische Katalyse zugängliche Alkanole sind solche obiger Formel (I) worin
- n: für einen ganzzahligen Wert von 0 bis 5 steht;
- Cyc: für einen gegebenenfalls substituierten, ein- oder mehrkernigen, gesättigten oder ungesättigten, carbocyclischen Ring steht, und
- R¹: für Halogen, SH, OH, NO₂, NR²R³ oder NR²R³R⁴⁺X⁻ steht, wobei R², R³ und R⁴ unabhängig voneinander für H oder einen Niedrigalkyl- oder Niedrigalkoxy-Rest stehen und X⁻ für ein Gegenion steht.

Die zur enzymatischen Synthese verwendeten Alkanole obiger Formel II sind an sich bekannte Verbindungen und unter Anwendung allgemein bekannter organischer Syntheseverfahren zugänglich (vgl. z.B. EP-A- 0 273 658).

Vorzugsweise steht n in obigen Verbindungen für 0, 1 oder 2, insbesondere für 1.

Als Beispiele für carbocyclische Gruppen Cyc sind insbesondere ein- oder zweikernige, vorzugsweise einkernige zu nennen:

Diese carbocyclischen Ringe umfassen insbesondere 3 bis 12, vorzugsweise 4, 5 oder 6 Ring-Kohlenstoffatomen. Als Beispiele können genannt werden Cyclopropyl, Cyclobutyl, Cyclopenty, Cyclohexyl, Cycloheptyl, die ein- oder mehrfach ungesättigten Analoga davon, wie Cyclobutenyl, Cyclopentenyl, Cyclohexenyl, Cycloheptenyl, Cyclohexadienyl, Cycloheptadienyl.

Die Reste Cyc können dabei über eine beliebige Ringposition an das Alkanon bzw. das Alkanol gebunden sein.

Die Reste Cyc können weiterhin ein- oder mehrfach, wie z.B. ein- oder zweifach, substituiert sein. Vorzugsweise sitzen die Substituenten an einem Ring-Kohlenstoffatom. Beispiele für geeignete Substituenten sind Halogen, Niedrigalkyl, Niedrigalkenyl, Niedrigalkoxy, -OH, -SH, -NO₂ oder NR²R³, wobei R² und R³ obige Bedeutungen besitzen, bevorzugt Halogen oder Niedrigalkyl.

R¹ steht für Halogen, NR²R³ oder NR²R³R⁴⁺X⁻, wobei R², R³ bzw. R², R³ und R⁴ unabhängig voneinander für H oder einen Niedrigalkyl- oder Niedrigalkoxy-Rest stehen und X⁻ für ein Gegenion steht, wobei vorzugsweise einer der Reste R², R³ und R⁴ für H steht. Geeignete Gegenionen sind beispielsweise Säureanionen, wie sie beispielsweise bei Herstellung eines Säureadditionssalzes anfallen. Beispiel hierzu sind z.B. in der EP-A-0 273 658 genannt, worauf hiermit Bezug genommen wird. Bevorzugte Beispiele für Reste R¹ sind insbesondere Fluor oder Chlor, sowie NR²R³ worin R² und R³ gleich oder verschieden sind und für H oder Methyl, Ethyl oder n-Propyl stehen; besonders bevorzugt steht R¹ für Chlor oder -NHMethyl.

### C. Enzyme mit Dehydrogenase-Aktivität

Die erfindungsgemäßen Enzyme mit Dehydrogenasen-Aktivität lassen sich in Mikroorganismen der Gattung Candida finden. Das Enzym bzw. die Enzyme besitzen eine hohe enzymatische Aktivität zur Reduktion von Alkanonen der Formel **II.** Andere Substrate, wie z.B. die Dimethylderivate des Ketons werden wie die Monomethylverbindungen ebenfalls von der Dehyrdogenase umgesetzt.

Ohne darauf beschränkt zu sein sind solche Enzyme bevorzugt aus Mikroorganismen der Gattungen Candida, insbesondere der Art C. magnoliae zugänglich.

Bevorzugte Enzym mit Dehydrogenase-Aktivität umfassen eine Aminosäuresequenz gemäß SEQ ID NO: 2 oder 4 oder eine Aminosäuresequenz, bei der bis zu 25%, bevorzugt bis zu 20%, besonders bevorzugt bis zu 10%, insbesondere bis zu 8,6,5,4,3,2, und 1 % der Aminosäurereste gegenüber SEC ID NO: 2 oder NO:4 durch Deletion; Insertion, Substitution oder einer Kombination davon verändert sind.

Besonders bevorzugt sind solche der o.g. Dehydrogenasen, die in Position 2 einen Thr-Rest und oder in Position 12 einen Gly-Rest tragen.

Homologe der erfindungsgemäßen Proteine oder Polypeptide können durch Mutagenese erzeugt werden, z.B. durch Punktmutation oder Verkürzung des Proteins.

Homologe der erfindungsgemäßen Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden.

Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

### D. Kodierende Nukleinsäuresequenzen

Die Begriffe "exprimieren" oder "Überexpression" beschreiben im Kontext der Erfindung die Produktion bzw. Erhöhung der intrazellulären Aktivität eines oder mehrerer Enzyme in einem Mikroorganismus, die durch die entsprechende DNA kodiert werden. Dazu kann man beispielsweise ein Gen in einen Organismus einbringen, ein vorhandenes Gen durch ein anderes Gen ersetzen, die Kopienzahl des Gens bzw. der Gene erhöhen, einen starken Promotor verwenden oder ein Gen verwenden, das für ein entsprechendes Enzym mit einer hohen Aktivität kodiert und man kann gegebenenfalls diese Maßnahmen kombinieren.

Gegenstand der Erfindung sind insbesondere Nukleinsäuresequenzen, die für ein Enzym mit Dehydrogenase-Aktivität kodieren. Bevorzugt sind Nukleinsäuresequenzen umfassend eine Sequenz gemäß SEQ ID NO:1 oder NO:3. Alle hierin erwähnten Nukleinsäuresequenzen (einzel- und doppelsträngige DNA- und RNA-Sequenzen, wie z.B. cDNA und mRNA) sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Gegenstand der Erfindung sind auch Nukleinsäuresequenzen (einzel- und doppelsträngige DNA- und RNA-Sequenzen, wie z.B. cDNA und mRNA), kodierend für eines der obigen Polypeptide und deren funktionalen Äquivalenten, welche z.B. unter Verwendung künstlicher Nukleotidanaloga zugänglich sind.

Die Erfindung betrifft sowohl isolierte Nukleinsäuremolekole, welche für erfindungsgemäße Polypeptide bzw. Proteine oder biologisch aktive Abschnitte davon kodieren, als auch Nukleinsäurefragmente, die z.B. zur Verwendung als Hybridisierungssonden oder Primer zur Identifizierung oder Amplifizierung von erfindungsgemäßer kodierenden Nukleinsäuren verwendet werden können.

Die erfindungsgemäßen Nukleinsäuremoleküle können zudem untranslatierte Sequenzen vom 3'- und/oder 5'-Ende des kodierenden Genbereichs enthalten.

Die Erfindung umfasst weiterhin die zu den konkret beschriebenen Nukleotidsequenzen komplementären Nukleinsäuremoleküle oder einen Abschnitt davon.

Die erfindungsgemäßen Nukleotidsequenzen ermöglichen die Erzeugung von Sonden und Primern, die zur Identifizierung und/oder Klonierung homologer Sequenzen in anderen Zelltypen und Organismen verwendbar sind. Solche Sonden bzw. Primer umfassen gewöhnlich einen Nukleotidsequenzbereich, der unter "stringenten" Bedingungen (siehe unten) an mindestens etwa 12, vorzugsweise mindestens etwa 25, wie z.B. etwa 40, 50 oder 75 aufeinanderfolgende Nukleotide eines Sense-Stranges einer erfindungsgemäßen Nukleinsäuresequenz oder eines entsprechenden Antisense-Stranges hybridisiert.

Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein erfindungsgemäßes Nukleinsäuremolekül kann mittels molekularbiologischer Standard-Techniken und der erfindungsgemäß bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies lässt sich ein Nukleinsäuremolekül, umfassend eine der offenbarten Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die erfindungsgemäßen Oligonukleotide können ferner durch Standard-Syntheseverfahren, z.B. mit einem automatischen DNA-Synthesegerät, hergestellt werden.

Die erfindungsgemäßen Nukleinsäuresequenzen lassen sich prinzipiell aus allen Organismen identifizieren und isolieren. Vorteilhaft lassen sich die erfindungsgemäßen Nukleinsäuresequenzen, wie SEQ ID NO: 1 oder NO:3 aus Mikroorganismen der Gattung Candida isolieren.

Erfindungsgemäße Nukleinsäuresequenzen, wie SEQ ID NO: 1 oder NO:3 oder Derivate davon, Homologe oder Teile dieser Sequenzen, lassen sich beispielsweise mit üblichen Hybridisierungsverfahren oder der PCR-Technik aus anderen Pilzen oder Bakterien, z.B, über genomische oder cDNA-Banken, isolieren. Diese DNA-Sequenzen hybridisieren unter Standardbedingungen mit den erfindungsgemäßen Sequenzen. Zur Hybridisierung werden vorteilhaft kurze Oligonukleotide der konservierten Bereiche beispielsweise aus dem aktiven Zentrum, die über Vergleiche mit der L-Camitin Dehydrogenase in dem Fachmann bekannter Weise ermittelt werden können, verwendet. Es können aber auch längere Fragmente der erfindungsgemäßen Nukleinsäuren oder die vollständigen Sequenzen für die Hybridisierung verwendet werden. Je nach der verwendeten Nukleinsäure (Oligonukleotid, längeres Fragment oder vollständige Sequenz) oder je nachdem welche Nukleinsäureart DNA oder RNA für die Hybridisierung verwendet werden, variieren diese Standardbedingungen, So liegen beispielsweise die Schmelztemperaturen für DNA:DNA-Hybride ca 10 °C niedriger als die von DNA:RNA-Hybriden gleicher Länge.

Unter Standardbedingungen sind beispielsweise je nach Nukleinsäure Temperaturen zwischen 42 und 58 °C in einer wässrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50% Formamid wie beispielsweise 42 °C in 5 x SSC, 50% Formamid zu verstehen. Vorteilhafterweise liegen die Hybridisierungsbedingungen für DNA:DNA-Hybride bei 0,1 x SSC und Temperaturen zwischen etwa 20 °C bis 45 °C, bevorzugt zwischen etwa 30 °C bis 45 °C. Für DNA:RNA-Hybride liegen die Hybridisierungsbedingungen vorteilhaft bei 0,1 x SSC und Temperaturen zwischen etwa 30 °C bis 55 °C, bevorzugt zwischen etwa 45 °C bis 55 °C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik, wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Harnes and Higgins (eds), 1985, Nucleic Acids Hybridization: A Practical Approach, IRL Press at Oxford University Press, Oxford; Brown (ed), 1981, Essential Molecular Biology: A Practical Approach, IRL Press at Oxford University Press, Oxford.

Gegenstand der Erfindung sind auch Derivate der konkret offenbarten oder ableitbaren Nukleinsäuresequenzen.

So können weitere erfindungsgemäße Nukleinsäuresequenzen z.B. von SEQ ID NO:1 oder NO:3 abgeleitet sein und sich davon durch Addition, Substitution, Insertion oder Deletion einzelner oder mehrerer Nukleotide unterscheiden, aber weiterhin für Polypeptide mit dem gewünschten Eigenschaftsprofil kodieren.

Erfindungsgemäß umfasst sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon.

Gegenstand sind ebenso durch konservative Nukleotidsubstutionen (d.h. die betreffende Aminosäure wird durch eine Aminosäure gleicher Ladung, Größe, Polarität und/oder Löslichkeit ersetzt) erhältliche Sequenzen.

Gegenstand der Erfindung sind auch die durch Sequenzpolymorphismen von den konkret offenbarten Nukleinsäuren abgeleiteten Moleküle. Diese genetischen Polymorphismen können zwischen Individuen innerhalb einer Population aufgrund der natürlichen Variation existieren. Diese natürlichen Variationen bewirken üblicherweise eine Varianz von 1 bis 6% in der Nukleotidsequenz eines Gens.

Unter Derivaten der erfindungsgemäßen Nukleinsäuresequenz mit der Sequenz SEQ ID NO:1 oder NO:3 sind beispielsweise Allelvarianten zu verstehen, die mindestens 40% Homologie auf der abgeleiteten Aminosäureebene, bevorzugt mindestens 60% Homologie, ganz besonders bevorzugt mindestens 80% Homologie über den gesamten Sequenzbereich aufweisen (bezüglich Homologie auf Aminosäureebene sei auf obige Ausführungen zu den Polypeptiden verwiesen auf). Ober Teilbereiche der Sequenzen können die Homologien vorteilhaft höher liegen.

Weiterhin sind unter Derivate auch Homologe der erfindungsgemäßen Nukleinsäuresequenzen, insbesondere der SEQ ID NO:1 oder NO:3, beispielsweise pilzliche oder bakterielle Homologe, verkürzte Sequenzen, Einzelstrang-DNA oder RNA der kodierenden und nichtkodierenden DNA-Sequenz, zu verstehen. So besitzen z.B. Homologe zur der SEQ ID NO:1 auf DNA-Ebene eine Homologie von mindestens 40%, bevorzugt von mindestens 60%, besonders bevorzugt von mindestens 70%, ganz besonders bevorzugt von mindestens 80% über den gesamten in SEQ ID NO:1 angegebenen DNA-Bereich.

Außerdem sind unter Derivaten beispielsweise Fusionen mit Promotoren zu verstehen. Die Promotoren, die den angegebenen Nukleotidsequenzen vorgeschalten sind, können durch ein oder mehrere Nukleotidaustausche, Insertionen, Inversionen und/oder Deletionen verändert sein, ohne dass aber die Funktionalität bzw. Wirksamkeit der Promotoren beeinträchtigt sind. Des weiteren können die Promotoren durch Veränderung ihrer Sequenz in ihrer Wirksamkeit erhöht oder komplett durch wirksamere Promotoren auch artfremder Organismen ausgetauscht werden.

Unter Derivaten sind auch Varianten zu verstehen, deren Nukleotidsequenz im Bereich von -1 bis -1000 Basen stromaufwärts vor dem Startkodon oder 0 bis 1000 Basen stromabwärts nach dem Stopkodon so verändert wurden, dass die Genexpression und/oder die Proteinexpression verändert, bevorzugt erhöht wird.

Weiterhin umfasst die Erfindung auch Nukleinsäuresequenzen, welchen mit oben genannten kodierenden Sequenzen unter "stringenten Bedingungen" hybridisieren. Diese Polynukleotide lassen sich bei der Durchmusterung von genomischen oder cDNA-Banken auffinden und gegebenenfalls daraus mit geeigneten Primem mittels PCR vermehren und anschließend beispielsweise mit geeigneten Sonden isolieren. Darüber hinaus können erfindungsgemäße Polynukleotide auch auf chemischem Wege synthetisiert werden. Unter dieser Eigenschaft versteht man die Fähigkeit eines Poly- oder Oligonukleotids unter stringenten Bedingungen an eine nahezu komplementäre Sequenz zu binden, während unter diesen Bedingungen unspezifische Bindungen zwischen nicht-komplementären Partnern unterbleiben. Dazu sollten die Sequenzen zu 70-100%, vorzugsweise zu 90-100%, komplementär sein. Die Eigenschaft komplementärer Sequenzen, spezifisch aneinander binden zu können, macht man sich beispielsweise in der Northern- oder Southem-Blot-Technik oder bei der Primerbindung in PCR oder RT-PCR zunutze. Üblicherweise werden dazu Oligonukleotide ab einer Länge von 30 Basenpaaren eingesetzt. Unter stringenten Bedingungen versteht man beispielsweise in der Northern-Blot-Technik die Verwendung einer 50 -70 °C, vorzugsweise 60 - 65 °C warmen Waschlösung, beispielsweise 0,1 x SSC-Puffer mit 0,1% SDS (20x SSC: 3M NaCl, 0,3M Na-Citrat, pH 7,0) zur Elution unspezifisch hybridisierter cDNA-Sonden oder Oligonukleotide. Dabei bleiben, wie oben erwähnt, nur in hohem Maße komplementäre Nukleinsäuren aneinander gebunden. Die Einstellung stringenter Bedingungen ist dem Fachmann bekannt und ist z:B. in Ausubel et al., Current Protocols in Molecular Biology, John Wiley & Sons, N.Y. (1989), 6.3.1-6.3.6. beschrieben.

### E. Erfindungsgemäße Konstrukte

Gegenstand der Erfindung sind außerdem Expressionskonstrukte, enthaltend unter der genetischen Kontrolle regulativer Nukleinsäuresequenzen eine für ein erfindungsgemäßes Polypeptid kodierende Nukleinsäuresequenz; sowie Vektoren, umfassend wenigstens eines dieser Expressionskonstrukte.

Vorzugsweise umfassen solche erfindungsgemäßen Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz.

Unter einer "operativen Verknüpfung" versteht man die sequentielle Anordnung von Promotor, kodierender Sequenz, Terminator und gegebenenfalls weiterer regulativer Elemente derart, dass jedes der regulativen Elemente seine Funktion bei der Expression der kodierenden Sequenz bestimmungsgemäß erfüllen kann. Beispiele für operativ verknüpfbare Sequenzen sind Targeting-Sequenzen sowie Enhancer, Polyadenylierungssignale und dergleichen. Weitere regulative Elemente umfassen selektierbare Marker, Amplifikationssignale, Replikationsurspronge und dergleichen. Geeignete regulatorische Sequenzen sind z.B. beschrieben in Goeddel, Gene Expression Technology: Methods in Enzymology 185, Academic Press, San Diego, CA (1990).

Unter einem erfindungsgemäßen Nukleinsäurekonstrukt sind insbesondere die Dehydrogenasegene mit Sequenz SEQ ID NO:1 und NO:3 und die Derivate und Homologen davon zu verstehen, die mit einem oder mehreren Regulationssignalen vorteilhafterweise zur Steuerung, z.B. Erhöhung, der Genexpression operativ oder funktionell verknüpft wurden.

Zusätzlich zu diesen Regulationssequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Das Nukleinsäurekonstrukt kann aber auch einfacher aufgebaut sein, das heißt es wurden keine zusätzlichen Regulationssignale vor die kodierende Sequenz (wie z.B. SEQ ID NO:1 oder NO:3 oder seine Homologen) insertiert und der natürliche Promotor mit seiner Regulation wurde nicht entfernt. Stattdessen wird die natürliche Regulationssequenz so mutiert, dass keine Regulation mehr erfolgt und die Genexpression gesteigert wird.

Ein bevorzugtes Nukleinsäurekonstrukt enthält vorteilhafterweise auch eine oder mehrere der schon erwähnten "Enhancer" Sequenzen, funktionell verknüpft mit dem Promotor, die eine erhöhte Expression der Nukleinsäuresequenz ermöglichen. Auch am 3'-Ende der DNA-Sequenzen können zusätzliche vorteilhafte Sequenzen inseriert werden, wie weitere regulatorische Elemente oder Terminatoren. Die erfindungsgemäßen Nukleinsäuren können in einer oder mehreren Kopien im Konstrukt enthalten sein. Im Konstrukt können noch weitere Marker, wie Antibiotikaresistenzen oder Auxotrophien komplementierende Gene, gegebenenfalls zur Selektion auf das Konstrukt enthalten sein.

Vorteilhafte Regulationssequenzen für das erfindungsgemäße Verfahren sind beispielsweise in Promotoren wie cos-, tac-, trp-, tet-, trp-tet-, Ipp-, lac-, lpp-lac-, lacl^{q}-, T7-, T5-, T3-, gal-, trc-, ara-, rhaP (rhaP_{BAD})SP6-, lambda-P_{R}- oder im lambda-P_{L}-Promotor enthalten, die vorteilhafterweise in gram-negativen Bakterien Anwendung finden. Weitere vorteilhafte Regulationssequenzen sind beispielsweise in den gram-positiven Promotoren amy und SPO2, in den Hefe- oder Pilzpromotoren ADC1, MFalpha , AC, P-60, CYC1, GAPDH, TEF, rp28, ADH enthalten. In diesem Zusammenhang sind auch die Promotoren der Pyruvatdecarboxylase und der Methanoloxidase, beispielsweise aus Hansenula vorteilhaft. Es können auch künstliche Promotoren für die Regulation verwendet werden.

Das Nukieinsäurekonstrukt wird zur Expression in einem Wirtsorganismus vorteilhafterweise in einen Vektor, wie beispielsweise einem Plasmid oder einem Phagen inseriert, der eine optimale Expression der Gene im Wirt ermöglicht. Unter Vektoren sind außer Plasmiden und Phagen auch alle anderen dem Fachmann bekannten Vektoren, also z.B. Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden. Diese Vektoren stellen eine weitere Ausgestaltung der Erfindung dar. Geeignete Plasmide sind beispielsweise in E. coli pLG338, pACYC184, pBR322, pUC18, pUC19, pKC30, pRep4, pHS1, pKK223-3, pDHE19.2, pHS2, pPLc236, pMBL24, pLG200, pUR290, pIN-III¹¹³-81, □gt11 oder pBdCl, in Streptomyces plJ101, plJ364, plJ702 oder plJ361, in Bacillus pUB110, pC194 oder pBD214, in Corynebacterium pSA77 oder pAJ667, in Pilzen pALS1, plL2 oder pBB116, in Hefen 2alphaM, pAG-1, YEp6, YEp13 oder pEMBLYe23 oder in Pflanzen pLGV23, pGHlac⁺, pBIN19, pAK2004 oder ph51. Die genannten Plasmide stellen eine kleine Auswahl der möglichen Plasmide dar. Weitere Plasmide sind dem Fachmann wohl bekannt und können beispielsweise aus dem Buch Cloning Vectors (Eds. Pouwels P. H. et al. Elsevier, Amsterdam-New York-Oxford, 1985, ISBN 0 444 904018) entnommen werden.

Vorteilhafterweise enthält das Nukleinsäurekonstrukt zur Expression der weiteren enthaltenen Gene zusätzlich noch 3'- und/oder 5'-terminale regulatorische Sequenzen zur Steigerung der Expression, die je nach ausgewähltem Wirtorganismus und Gen oder Gene für eine optimale Expression ausgewählt werden.

Diese regulatorischen Sequenzen sollen die gezielte Expression der Gene und der Proteinexpression ermöglichen. Dies kann beispielsweise je nach Wirtsorganismus bedeuten, dass das Gen erst nach Induktion exprimiert oder überexprimiert wird, oder dass es sofort exprimiert und/oder überexprimiert wird.

Die regulatorischen Sequenzen bzw. Faktoren können dabei vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

In einer weiteren Ausgestaltungsform des Vektors kann der das erfindungsgemäße Nukleinsäurekonstrukt oder die erfindungsgemäße Nukleinsäure enthaltende Vektor auch vorteilhafterweise in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus dem Nukleinsäurekonstrukt oder der erfindungsgemäßen Nukleinsäure bestehen.

Für eine optimale Expression heterologer Gene in Organismen ist es vorteilhaft die Nukleinsäuresequenzen entsprechend des im Organismus verwendeten spezifischen "codon usage" zu verändern. Der "codon usage" lässt sich anhand von Computerauswertungen anderer, bekannter Gene des betreffenden Organismus leicht ermitteln.

Die Herstellung einer erfindungsgemäßen Expressionskassette erfolgt durch Fusion eines geeigneten Promotors mit einer geeigneten kodierenden Nukleotidsequenz sowie einem Terminator- oder Polyadenyllerungesignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1964) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden.

### F. Erfindungsgemäß brauchbare Wirte

Mit Hilfe der erfindungsgemäßen Vektoren sind rekombinante Mikroorganismen herstellbar, welche beispielsweise mit wenigstens einem erfindungsgemäßen Vektor transformiert sind und zur Produktion der erfindungsgemäßen Polypeptide eingesetzt werden können. Vorteilhafterweise werden die oben beschriebenen erfindungsgemäßen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning: A Laboratory Manual. 2. Aufl., Cold Spring Harbor Laboratory, Cold Spring Harbor Laboratory Press, Cold Spring Harbor, NY, 1989 beschrieben.

Erfindungsgemäß sind auch homolog rekombinierte Mikroorganismen herstellbar. Dazu wird ein Vektor hergestellt, der zumindest einen Abschnitt eines erfindungsgemäßen Gens oder einer kodierenden Sequenz enthält, worin gegebenenfalls wenigstens eine Aminosäure-Deletion, -Addition oder -Substitution eingebracht worden ist, um die erfindungsgemäße Sequenz zu verändern, z.B. funktionell zu disrumpieren ("Knockout"-Vektor). Die eingebrachte Sequenz kann z.B. auch ein Homologes aus einem verwandten Mikroorganismus sein oder aus einer Säugetier-, Hefe- oder Insektenquelle abgeleitet sein. Der zur homologen Rekombination verwendete Vektor kann alternativ derart ausgestaltet sein, dass das endogene Gen bei homologer Rekombination mutiert oder anderweitig verändert ist, jedoch noch das funktionelle Protein kodiert (z.B. kann der stromaufwärts gelegene regulatorische Bereich derart verändert sein, dass dadurch die Expression des endogenen Proteins verändert wird). Der veränderte Abschnitt des erfindungsgemäßen Gens ist im homologen Rekombinationsvektor. Die Konstruktion geeigneter Vektoren zur homologen Rekombination ist z.B. beschrieben in Thomas, K.R. und Capecchi, M.R. (1987) Cell 51:503.

Als rekombinante Wirtsorganismen für die erfindungsgemäße Nukleinsäure oder dem Nukleinsäurekonstrukt kommen prinzipiell alle prokaryontischen oder eukaryontischen Organismen in Frage. Vorteilhafterweise werden als Wirtsorganismen Mikroorganismen wie Bakterien, Pilze oder Hefen verwendet. Vorteilhaft werden gram-positive oder gram-negative Bakterien, bevorzugt Bakterien der Familien Enterobacteriaceae, Pseudomonadaceae, Rhizoblaceae, Streptomycetaceae oder Nocardiaceae, besonders bevorzugt Bakterien der Gattungen Escherichia, Pseudomonas, Streptomyces, Nocardia, Burkholderia, Salmonella, Agrobacterium oder Rhodococcus verwendet. Ganz besonders bevorzugt ist die Gattung und Art Escherichia coli. Weitere vorteilhafte Bakterien sind darüber hinaus in der Gruppe der alpha-Proteobacterien, beta-Proteobacterien oder gamma-Proteobacterien zu finden.

Der Wirtsorganismus oder die Wirtsorganismen gemäß der Erfindung enthalten dabei vorzugsweise mindestens eine der in dieser Erfindung beschriebenen Nukleinsäuresequenzen, Nukleinsäurekonstrukte oder Vektoren, die für ein Enzym mit L-Camitin Dehydrogenaseaktivität kodieren (Kleber HP (1997) FEMS Microbiology, 147, 1 - 9).

Die im erfindungsgemäßen Verfahren verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0 °C und 100 °C, bevorzugt zwischen 10 °C bis 60 °C unter Sauerstoffbegasung angezogen. Dabei kann der pH der Nährflüssigkeit auf einen festen Wert gehalten werden, das heißt während der Anzucht reguliert werden oder nicht. Die Anzucht kann "batch"-weise, "semi batch"-weise oder kontinuierlich erfolgen. Nährstoffe können zu beginn der Fermentation vorgelegt oder semikontinuierlich oder kontinuierlich nachgefüttert werden. Das Keton kann direkt zur Anzucht gegeben werden oder vorteilhaft nach Anzucht. Die Enzyme können nach dem in den Beispielen beschriebe- , nen Verfahren aus den Organismen isoliert werden oder als Rohextrakt für die Reaktion verwendet werden.

Die Wirtsorganismen enthalten vorteilhaft 1 U/l Enzymaktivität, wie z.B. L-Camitinedehydrogenaseaktivität, bevorzugt 100 U/l, besonders bevorzugt mehr als 1000 U/l.

### G. Rekombinante Herstellung der Polypeptide:

Gegenstand der Erfindung sind weiterhin Verfahren zur rekombinanten Herstellung erfindungsgemäße Polypeptide oder funktioneller, biologisch aktiver Fragmente davon, wobei man einen Polypeptide-produzierenden Mikroorganismus kultiviert, gegebenenfalls die Expression der Polypeptide induziert und diese aus der Kultur isoliert. Die Polypeptide können so auch in großtechnischem Maßstab produziert werden, falls dies erwünscht ist.

Der rekombinante Mikroorganismus kann nach bekannten Verfahren kultiviert und fermentiert werden. Bakterien können beispielsweise in TB- oder LB-Medium und bei einer Temperatur von 20 bis 40 °C und einem pH-Wert von 6 bis 9 vermehrt werden. Im Einzelnen werden geeignete Kultivierungsbedingungen beispielsweise in T. Maniatis, E.F. Fritsch and J. Sambrook, Molecular Cloning: A Laboratory Manual, Cold Spring Harbor Laboratory, Cold Spring Harbor, NY (1889) beschrieben.

Die Zellen werden dann, falls die Polypeptide nicht in das Kulturmedium sezerniert werden, aufgeschlossen und das Produkt nach bekannten Proteinisolierungsverfahren aus dem Lysat gewonnen. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren ausgeschlossen werden.

Eine Aufreinigung der Polypeptide kann mit bekannten, chromatographischen Verfahren erzielt werden, wie Molekularsieb-Chromatographie (Gelfiltration), wie Q-Sepharose-Chromatographie, Ionenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, F. G., Biochemische Arbeitsmethoden, Verlag Walter de Gruyter, Berlin, New York oder in Scopes, R., Protein Purification, Springer Verlag, New York, Heidelberg, Berlin beschrieben.

Vorteilhaft kann es sein, zur Isolierung des rekombinanten Proteins Vektorsysteme oder Oligonukleotide zu verwenden, die die cDNA um bestimmte Nukleotidsequenzen verlängern und damit für veränderte Polypeptide oder Fusionsproteine kodieren, die z.B. einer einfacheren Reinigung dienen. Derartige geeignete Modifikationen sind beispielsweise als Anker fungierende sogenannte "Tags", wie z.B. die als Hexa-Histidin-Anker bekannte Modifikation oder Epitope, die als Antigene von Antikörpern erkannt werden können (beschrieben zum Beispiel in Harlow, E. and Lane, D., 1888, Antibodies: A Laboratory Manual. Cold Spring Harbor (N.Y.) Press). Diese Anker können zur Anheftung der Proteine an einen festen Träger, wie z.B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig können diese Anker auch zur Erkennung der Proteine verwendet werden. Zur Erkennung der Proteine können außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Proteine verwendet werden.

H. Durchführung des erfindungsgemäßen Verfahrens zur Herstellung von (S)-Alkanolen

Die Enzyme mit Dehydrogenase-Aktivität können im erfindungsgemäßen Verfahren als freies oder immobilisiertes Enzym verwendet werden.

Das erfindungsgemäße Verfahren wird vorteilhaft bei einer Temperatur zwischen 0 °C bis 95 °C, bevorzugt zwischen 10 °C bis 85 °C, besonders bevorzugt zwischen 15 °C bis 75 °C durchgeführt.

Der pH-Wert im erfindungsgemäßen Verfahren wird vorteilhaft zwischen pH 4 und 12, bevorzugt zwischen pH 4,5 und 9, besonders bevorzugt zwischen pH 5 und 8 gehalten.

Unter enantiomerenreinen bzw. chiralen Produkten sind im erfindungsgemäßen Verfahren Enantiomere zu verstehen, die eine Enantiomerenanreicherung zeigen. Bevorzugt werden im Verfahren Enantiomerenreinheiten von mindestens 70%ee, bevorzugt von min. 80%ee, besonders bevorzugt von min. 90%ee, ganz besonders bevorzugt min. 98%ee erreicht.

Für das erfindungsgemäße Verfahren können wachsende Zellen verwendet werden, die die erfindungsgemäßen Nukleinsäuren, Nukleinsäurekonstrukte oder Vektoren enthalten. Auch ruhende oder aufgeschlossene Zellen können verwendet werden. Unter aufgeschlossenen Zellen sind beispielsweise Zellen zu verstehen, die über eine Behandlung mit beispielsweise Lösungsmitteln durchlässig gemacht worden sind, oder Zellen die Ober eine Enzymbehandlung, über eine mechanische Behandlung (z.B. French Press oder Ultraschall) oder über eine sonstige Methode aufgebrochen wurden.

Die so erhaltenen Rohextrakte sind für das erfindungsgemäße Verfahren vorteilhaft geeignet. Auch gereinigte oder angereinigte Enzyme können für das Verfahren verwendet werden. Ebenfalls geeignet sind immobilisierte Mikroorganismen oder Enzyme, die vorteilhaft in der Reaktion Anwendung finden können.

Werden für das erfindungsgemäße Verfahren freie Organismen oder Enzyme verwendet, so werden diese vor der Extraktion zweckmäßigerweise abgetrennt beispielsweise über eine Filtration oder Zentrifugation.

Das im erfindungsgemäßen Verfahren hergestellte Produkt lässt sich vorteilhaft aus der wässrigen Reaktionslösung über Extraktion oder Destillation oder vorteilhaft über Extraktion und Destillation gewinnen. Die Extraktion kann zur Erhöhung der Ausbeute mehrfach wiederholt werden. Beispiele für geeignete Extraktionsmittel sind Lösungsmittel, wie Toluol, Methylenchlorid, Butylacetyt, Diisopropylether, Benzol, MTBE oder Essigester, ohne darauf beschränkt zu sein.

Nach Einengen der organischen Phase können die Produkte in der Regel in guten chemischen Reinheiten, das heißt größer 80 % chemische Reinheit, gewonnen werden. Nach Extraktion kann die organische Phase mit dem Produkt aber auch nur zum Teil eingeengt werden und das Produkt auskristallisiert werden. Dazu wird die Lösung vorteilhaft auf eine Temperatur von 0 °C bis 10 °C abgekühlt. Die Kristallisation kann auch direkt aus der organischen Lösung oder aus einer wässrigen Lösung erfolgen. Das auskristallisierte Produkt kann nochmals im gleichen oder in einem anderen Lösungsmittel zur erneuten Kristallisation aufgenommen werden und nochmals kristallisiert werden. Durch die anschließende vorteilhafte mindestens einmalig durchgeführte Kristallisation kann die Enantiomerenreinheit des Produktes falls erforderlich weiter gesteigert werden.

Bei den genannten Aufarbeitungsarten lässt sich das Produkt des erfindungsgemäßen Verfahrens in Ausbeuten von 60 bis 100%, bevorzugt von 80 bis 100%, besonders bevorzugt von 90 bis 100%, bezogen auf das für die Reaktion eingesetzte Substrat, wie z.B. von 3-Methylamino-1-(2-thienyl)-propan-1-on, isolieren. Das isolierte Produkt zeichnet sich durch eine hohe chemische Reinheit von > 90%, bevorzugt > 96% besonders bevorzugt von > 98% aus. Weiterhin haben die Produkt eine hohe Enantiomerenreinheit, die vorteilhaft falls erforderlich durch die Kristallisation weiter gesteigert werden kann.

Das erfindungsgemäße Verfahren kann batchweise, semi-batchweise oder kontinuierlich betrieben werden.

Die Durchführung des Verfahrens kann vorteilhafterweise in Bioreaktoren erfolgen, wie z.8. beschrieben in Biotechnology, Band 3, 2. Auflage, Rehm et al Hrsg., (1993) insbesondere Kapitel II.

Die obige Beschreibung und die nachstehenden Beispiele dienen nur der Verdeutlichung der Erfindung. Die für den Fachmann offensichtlichen, zahlreich möglichen Abwandlungen sind erfindungsgemäß ebenfalls umfasst.

### Experimenteller Teil:

### Beispiel 1

### Relative Aktivität der erfindungsgemäßen Dehydrogenasen gegenüber verschiedenen Substraten (in Klammern ist die optische Reinheit in %ee angegeben)

| Substrat | Enzym ADH-3 SEQ ID NO:2 | Enzym ADH-4 SEQ ID NO-4 | Vergleichsenzym EP 1152054 SEQ ID NO:1 |
|---|---|---|---|
| Acetophenon | 16,08 (>99%) | 3.14(70%) | 0,84 (>99%) |
| m-DICAP | 100 (>99%) | 56,38 (12%) | 2,3 (>99%) |
| CMAP | 34,46 (90%) | 24,89 (88%) | 29,44 (98%) |

Die Reaktion wurde ohne Cofaktor-Regenerierung durchgeführt.

### Reaktionsansatz:

180 µl 50 mM Kpi Puffer, enthaltend 1 mM MgCl2
20 µl Enzym-lösung
200 µl NADPH 10M Stammlösung
100 µl Substrat 1 M Stammlösung
500 µl Hexan

Das Reaktionsgemisch wurde bei 37°C ca. 20 Stunden bei 1000 U/min inkubiert. Anschließend wurde bei 20°C zentrifugiert, der klare Überstand entfernt und durch chirale Gaschromatographie analysiert.

### Beispiel 2

Relative Aktivität der erfindungsgemäßen Dehydrogenasen gegenüber verschiedenen Substraten (in Klammern ist die optische Reinheit in %ee angegeben) mit Cofaktor-Regenerierung.

Reaktionsdurchführung analog zu Beispiel 1. Die Cofaktorregenerierung wurde durch Zugabe von Glucose und Glucosedehydrogenase in einem ein-phasigen flüssigen System durchgeführt.

| Substrat | Enzym ADH-3 SEQ ID NO:2 | Enzym ADH-4 SEQ ID NO:4 | Vergleichsenzym EP 1152054 SEQ ID NO:1 |
|---|---|---|---|
| Acetophenon | 8,9 (>99% S) | nicht durchgeführt | 0,7 (>99% S) |
| m-DICAP | 100(>99%R) | 55,8(25% S) | 47,1 (83% R) |
| p-DICAP | 18,9 (>99% R) | 62,1 (44% S) | 62,5 (>99% R) |
| FCA | 99,5 (>99% R) | 53,6 (58% S) | 50,4 (>99% R) |

### Beispiel 3

Relative Aktivität der erfindungsgemäßen Dehydrogenasen, gegenüber verschiedenen Substraten (in Klammern ist die optische Reinheit in %ee angegeben) mit Cofaktor-Regenerierung im 2 Phasen-System.

Reaktionsdurchführung analog zu Beispiel 2. Die Cofaktorregenerierung wurde durch Zugabe von Glucose und Glucosedehydrogenase in einem zwei-phasigen flüssigen System (MTBE:wässrig) durchgeführt.

| Substrat | Enzym ADH-3 SEQ ID NO:2 | Enzym ADH-4 SEQ ID NO:4 | Vergleichsenzym EP 1152054 SEQ ID NO:1 |
|---|---|---|---|
| Acetophenon | 71,2 (>99% S) | 0,4 (70% S) | 0,2 (>99% S) |
| m-DICAP | 100 (>99% R ) | 24,6 (6% S) | 14 (>99% R) |
| p-DICAP | 30,4 (>99% R) | 27,6 (42% S) | 24,2 (>99% R) |
| FCA | 99,5 (>99% R) | 15,3(46%S) | 12,6 (>99% R) |
| CMAP | 100 (>99% R) | 77,1 (97% S) | 46,8 (>99% R) |

### Abkürzungen:

m-DICAP: 2,3'-Dichlor-acetophenon
p-DICAP: 2,4'-Dichlor-acetophenon
FCA: 4'-Fluor-2-Chlor-acetophenon
CMAP: 4-Chlor-methoxy-acetophenon

## Patentansprüche

1. Verfahren zur mikrobiologischen Herstellung von substituierten Alkanolen der Formel I 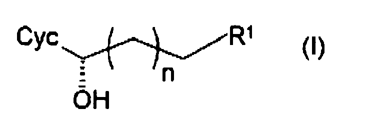 worin
n für einen ganzzahligen Wert von 0 bis 5 steht;
Cyc für einen gegebenenfalls substituierten, ein- oder mehrkernigen, gesättigten oder ungesättigten, carbocyclischen Ring steht, und
R¹ für Halogen, SH, OH, NO₂, NR²R³ oder NR²R³R⁴⁺X⁻ steht, wobei R², R³ und R⁴ unabhängig voneinander für H oder einen Niedrigalkyl- oder Niedrigal- koxy-Rest stehen und X⁻ für ein Gegenion steht,
wobei man in einem Alkanon der Formel II 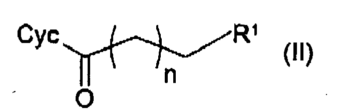 worin n, Cyc und R¹ die oben angegebenen Bedeutungen besitzen,
enthaltenden Medium,
a) einen eine Dehydrogenase mit der Polypeptidsequenz SEQ ID NO:2 oder NO:4, oder mit einer Polypeptidsequenz; bei der bis zu 25% der Aminosäurereste gegenüber SEQ ID NO: 2 oder NO:4 durch Deletion; Insertion, Substitution oder einer Kombination davon verändert sind, produzierenden Mikroorganismus kultiviert, oder
b) eine Dehydrogenase wie unter a) genannt inkubiert,
wobei die Verbindung der Formel II zur Verbindung der Formel I enzymatisch reduziert wird, und man das gebildete Produkt isoliert.

2. Verfahren nach einem der vorhergehenden Ansprüche, wobei das Enzym mit Dehydrogenase-Aktivität ausgewählt ist unter Enzymen, welche eine Aminosäuresequenz gemäß SEQ ID NO: 2 oder 4 umfassen, bei der bis zu 25% der Aminosäurereste durch Deletion; Insertion, Substitution oder einer Kombination davon verändert sind und welche Dehydrogenase-Aktivität besitzen und die enantioselektive Synthese einer Verbindung der Formel I katalysieren.

3. Verfahren nach einem der vorhergehenden Ansprüche, wobei das Enzym mit Dehydrogenase-Aktivität von einem Nukleinsäuresequenz gemäß SEQ ID NO:1 oder 3 kodiert wird, bei der bis zu 25% der Aminosäurereste durch Deletion; Insertion, Substitution oder einer Kombination davon verändert sind.

4. Verfahren nach einem der vorhergehenden Ansprüche, wobei man die Umsetzung unter Zugabe von Reduktionsäquivalenten oder die bei der Umsetzung verbrauchten Reduktionsäquivalente regenerierenden Bedingungen durchführt.

5. Verfahren nach einem der vorhergehenden Ansprüche, wobei die Umsetzung der Verbindung der Formel II in Gegenwart eines Mikroorganismus erfolgt, der ausgewählt ist unter Bakterien der Familien Enterobacteriaceae, Pseudomonadaceae, Rhizobiaceae, Streptomycetaceae und Nocardiaceae.

6. Verfahren nach einem der vorhergehenden Ansprüche, wobei der Mikroorganismus ein rekombinanter Mikroorganismus ist, der mit einem Nukleinsäurekonstrukt transformiert ist, welche für ein Enzym mit Dehydrogenase-Aktivität gemäß der Definition in einem der Ansprüche 2 bis 5 kodiert.

7. Polypeptid, welches eine Aminosäuresequenz gemäß SEQ ID NO: 2 oder 4 umfassen, bei der bis zu 25% der Aminosäurereste durch Deletion; Insertion, Substitution oder einer Kombination davon verändert sind und welche Dehydrogenase-Aktivität besitzen und die enantioselektive Synthese einer Verbindung der Formel I katalysieren.

8. Nukleinsäuresequenz, umfassend die kodierende Sequenz für ein Polypeptid gemäß Anspruch 7.

9. Expressionskassette, umfassend in operativer Verknüpfung mit wenigstens einer regulativen Nukleinsäuresequenz eine Nukleinsäuresequenz nach Anspruch 8.

10. Rekombinanter Vektor, umfassend wenigstens eine Expressionskassette nach Anspruch 9.

11. Prokaryotischer oder eukaryotischer Wirt, transformiert mit wenigstens einem Vektor nach Anspruch 10.

12. Verwendung eines Enzyms mit Dehydrogenase-Aktivität gemäß Anspruch 7 zur Herstellung von Verbindungen der Formel I.

## Claims

1. A method for the microbiological preparation of substituted alkanols of the formula I 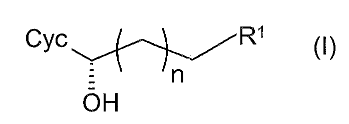 in which
n is an integer from 0 to 5;
Cyc is an unsubstituted or substituted, mono- or polynuclear, saturated or unsaturated, carbocyclic ring, and
R¹ is halogen, SH, OH, NO₂, NR²R³ or NR²R³R⁴⁺X⁻, where R², R³ and R⁴ independently of one another are H or a lower alkyl or lower alkoxy radical and X⁻ is a counterion,
wherein, in a medium comprising an alkanone of the formula II 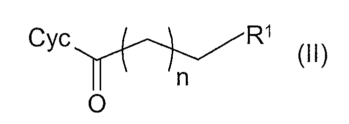 in which n, Cyc and R¹ are as defined above,
a) a microorganism producing a dehydrogenase having the polypeptide sequence SEQ ID NO: 2 or NO: 4, or having a polypeptide sequence in which up to 25% of the amino acid residues have been altered by deletion, insertion, substitution or a combination thereof, compared to SEQ ID NO: 2 or NO: 4 is cultured, or
b) a dehydrogenase as mentioned under a) is incubated,
the compound of the formula II being enzymatically reduced to give the compound of the formula I, and the product formed is isolated.

2. The method according to the preceding claim, wherein the enzyme having dehydrogenase activity is selected from among enzymes comprising an amino acid sequence according to SEQ ID NO: 2 or 4, in which up to 25 0 of the amino acid residues have been altered by deletion, insertion, substitution or a combination thereof, and which have dehydrogenase activity and catalyze the enantioselective synthesis of a compound of the formula I.

3. The method according to either of the preceding claims, wherein the enzyme having dehydrogenase activity is encoded by a nucleic acid sequence according to SEQ ID NO:1 or 3, in which up to 25% of the amino acid residues have been altered by deletion, insertion, substitution or a combination thereof.

4. The method according to any of the preceding claims, wherein the reaction is carried out with addition of reduction equivalents or under conditions in which the reduction equivalents consumed are regenerated.

5. The method according to any of the preceding claims, wherein the compound of the formula II is reacted in the presence of a microorganism selected from among the bacteria of the families Enterobacteriaceae, Pseudomonadaceae, Rhizobiaceae, Streptomycetaceae and Nocardiaceae.

6. The method according to any of the preceding claims, wherein the microorganism is a recombinant microorganism which has been transformed with a nucleic acid construct coding for an enzyme having dehydrogenase activity as defined in any of claims 2 to 5.

7. A polypeptide which comprises an amino acid sequence according to SEQ ID NO: 2 or 4, in which up to 25% of the amino acid residues have been altered by deletion, insertion, substitution or a combination thereof, and which has dehydrogenase activity and catalyzes the enantioselective synthesis of a compound of the formula I.

8. A nucleic acid sequence comprising the sequence coding for a polypeptide according to claim 7.

9. An expression cassette, comprising a nucleic acid sequence according to claim 8, which sequence is operatively linked to at least one regulatory nucleic acid sequence.

10. A recombinant vector, comprising at least one expression cassette according to claim 9.

11. A prokaryotic or eukaryotic host, transformed with at least one vector according to claim 10.

12. The use of an enzyme having dehydrogenase activity according to claim 7 for preparing compounds of the formula I.

## Revendications

1. Procédé de préparation microbiologique d'alcanols substitués de formule I 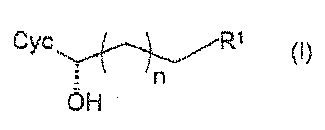 dans laquelle
n est un nombre entier de 0 à 5 ;
Cyc représente un noyau carbocyclique, saturé ou insaturé, mono- ou polycyclique, éventuellement substitué, et
R¹ est un halogène, SH, OH, NO₂, NR²R³ ou NR²R³R⁴⁺X⁻, où R², R³ et R⁴ représentent chacun indépendamment des autres H ou un radical alkyle inférieur ou alcoxy inférieur, et X⁻ est un contre-ion,
procédé dans lequel on cultive, dans un milieu contenant une alcanone de formule II 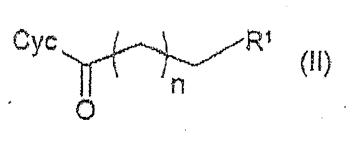 dans laquelle n, Cyc et R¹ ont les significations données ci-dessus,
a) un microorganisme produisant une déshydrogénase ayant la séquence polypeptidique SEQ ID N°2 ou 4, ou ayant une séquence polypeptidique dans laquelle jusqu'à 25 % des résidus d'acides aminés sont modifiés par délétion, insertion, substitution ou par une combinaison de celles-ci, par rapport à SEQ ID N°2 ou 4, ou
b) on incube une déshydrogénase telle que mentionnée en a),
le composé de formule II étant réduit par voie enzymatique en le composé de formule I et on isole le produit formé.

2. Procédé selon l'une des revendications précédentes, dans lequel l'enzyme ayant une activité de déshydrogénase est choisie parmi les enzymes qui comprennent une séquence d'acides aminés selon SEQ ID N°2 ou 4, dans laquelle jusqu'à 25 % des résidus d'acides aminés sont modifiés par délétion, insertion, substitution ou par une combinaison de celles-ci, et qui possèdent une activité de déshydrogénase, et qui catalysent la synthèse énantio-sélective d'un composé de formule I.

3. Procédé selon l'une des revendications précédentes, dans lequel l'enzyme ayant une activité de déshydrogénase est codée par une séquence d'acides nucléiques selon SEQ ID N°1 ou 3, dans laquelle jusqu'à 25 % des résidus d'acides aminés sont modifiés par délétion, insertion, substitution ou par une combinaison de celles-ci.

4. Procédé selon l'une des revendications précédentes, dans lequel on met en oeuvre la réaction par addition d'équivalents de réduction, ou dans des conditions qui régénèrent les équivalents de réduction consommés lors de la réaction.

5. Procédé selon l'une des revendications précédentes, dans lequel la réaction du composé de formule II a lieu en présence d'un microorganisme qui est choisi parmi les bactéries des familles Enterobacteriaceae, Pseudomonadaceae, Rhizobiaceae, Streptomycetaceae et Nocardiaceae.

6. Procédé selon l'une des revendications précédentes, dans lequel le microorganisme est un microorganisme recombinant, qui est transformé avec une construction d'acides nucléiques, qui code pour une enzyme ayant une activité de déshydrogénase selon la définition donnée dans l'une des revendications 2 à 5.

7. Polypeptide qui comprend une séquence d'acides aminés selon SEQ ID N°2 ou 4, dans laquelle jusqu'à 25 % des résidus d'acides aminés sont modifiés par délétion, insertion, substitution ou par une combinaison de celles-ci, et qui possèdent une activité de déshydrogénase, et qui catalysent la synthèse énantio-sélective d'un composé de formule I.

8. Séquences d'acides nucléiques comprenant la séquence codant pour un polypeptide selon la revendication 7.

9. Cassette d'expression comprenant, en liaison opérationnelle avec au moins une séquence d'acides nucléiques régulatrice, une séquence d'acides nucléiques selon la revendication 8.

10. Vecteur recombinant comprenant au moins une cassette d'expression selon la revendication 9.

11. Hôte procaryote ou eucaryote, transformé par au moins un vecteur selon la revendication 10.

12. Utilisation d'une enzyme ayant une activité de déshydrogénase selon la revendication 7 pour préparer des composés de formule I.


### SEQUENCE LISTING

<110>< BASF Aktiengesellschaft
<120> Neue Dehydrogenasen, deren Derivate und ein Verfahren zur Herstellung von optisch aktiven Alkanolen
<130> PF 57135
<160> 4
<170> PatentIn version 3.1
<210> 1
   <211> 723
   <212> DNA
   <213> Candida magnoliae
<220>
   <221> CDS
   <222> (1) .. (723)
   <223>
<400> 1
   atg acg act act tca aat gcg ctc gtc act gga ggc agc cgc ggc att        48
   Met Thr Thr Thr Ser Asn Ala Leu Val Thr Gly Gly Ser Arg Gly Ile
   1               5                   10                  15
   ggc gct gct tcc gcc att aag ctg gct cag gag ggc tac agt gtt acg        96
   Gly Ala Ala Ser Ala Ile Lys Leu Ala Gln Glu Gly Tyr Ser Val Thr
               20                  25                  30
   ctg gcc tct cgc agt gtt gat aaa ctg aat gaa gta aag gcg aaa ctc       144
   Leu Ala Ser Arg Ser Val Asp Lys Leu Asn Glu Val Lys Ala Lys Leu
           35                  40                  45
   cca att gta cag gac ggg cag aag cac tac att tgg gaa ctc gat ctg       192
   Pro Ile Val Gln Asp Gly Gln Lys His Tyr Ile Trp Glu Leu Asp Leu
       50                  55                  60
   gct gat gtg gaa gct gct tcg tcg ttc aag ggt gct cct ttg cct gct       240
   Ala Asp Val Glu Ala Ala Ser Ser Phe Lys Gly Ala Pro Leu Pro Ala
   65                  70                  75                  80
   agc agc tac gac gtc ttc gtt tcg aac gcg ggc gtc gct gcg ttc tcg       288
   Ser Ser Tyr Asp Val Phe Val Ser Asn Ala Gly Val Ala Ala Phe Ser
                   85                  90                  95
   ccc aca gcc gac cac gat gat aag gag tgg cag aac ttg ctc gcc gtg       336
   Pro Thr Ala Asp His Asp Asp Lys Glu Trp Gln Asn Leu Leu Ala Val
               100                 105                 110
   aac ttg tcg tcg ccc att gcc ctc acg aag gcc ctc ttg aag gac gtc       384
   Asn Leu Ser Ser Pro Ile Ala Leu Thr Lys Ala Leu Leu Lys Asp Val
           115                 120                 125
   tcc gaa agg cct gcg gac aat ccg ttg cag att atc tac att tcg tcg      432
   Ser Glu Arg Pro Ala Asp Asn Pro Leu Gln Ile Ile Tyr Ile Ser Ser
       130                 135                 140
   gtg gcc ggc ttg cat ggc gcc gcg cag gtc gcc gtg tac agt gca tct      480
   Val Ala Gly Leu His Gly Ala Ala Gln Val Ala Val Tyr Ser Ala Ser
   145                 150                 155                 160
   aag gcc ggt ctt gat ggt ttt atg cgc tcc gtc gcc cgt gag gtg ggc      528
   Lys Ala Gly Leu Asp Gly Phe Met Arg Ser Val Ala Arg Glu Val Gly
                   165                 170                 175
   ccg aag ggc atc cat gtg aac tcc atc aac ccc gga tac acc aag act      576
   Pro Lys Gly Ile His Val Asn Ser Ile Asn Pro Gly Tyr Thr Lys Thr
               180                 185                 190
   gaa atg acc gcg ggc att gaa gcc ctg cct gat ttg cct atc aag ggg      624
   Glu Met Thr Ala Gly Ile Glu Ala Leu Pro Asp Leu Pro Ile Lys Gly
           195                 200                 205
   tgg atc gag ccc gag gca att gct gac gcg gtt ctg ttt ctg gca aag      672
   Trp Ile Glu Pro Glu Ala Ile Ala Asp Ala Val Leu Phe Leu Ala Lys
       210                 215                 220
   tcc aag aat atc acc ggc aca aac att gtg gtc gac aat ggc ttg att      720
   Ser Lys Asn Ile Thr Gly Thr Asn Ile Val Val Asp Asn Gly Leu Ile
   225                 230                 235                 240
   gct                                                                  723
   Ala
<210> 2
   <211> 241
   <212> PRT
   <213> Candida magnoliae
<400> 2
   Met Thr Thr Thr Ser Asn Ala Leu Val Thr Gly Gly Ser Arg Gly Ile
   1               5                   10                  15
   Gly Ala Ala Ser Ala Ile Lys Leu Ala Gln Glu Gly Tyr Ser Val Thr
               20                  25                  30
   Leu Ala Ser Arg Ser Val Asp Lys Leu Asn Glu Val Lys Ala Lys Leu
           35                  40                  45
   Pro Ile Val Gln Asp Gly Gln Lys His Tyr Ile Trp Glu Leu Asp Leu
       50                  55                  60
   Ala Asp Val Glu Ala Ala Ser Ser Phe Lys Gly Ala Pro Leu Pro Ala
   65 70 75 80
   Ser Ser Tyr Asp Val Phe Val Ser Asn Ala Gly Val Ala Ala Phe Ser
   85 90 95
   Pro Thr Ala Asp His Asp Asp Lys Glu Trp Gln Asn Leu Leu Ala Val
   100 105 110
   Asn Leu Ser Ser Pro Ile Ala Leu Thr Lys Ala Leu Leu Lys Asp Val
   115 120 125
   Ser Glu Arg Pro Ala Asp Asn Pro Leu Gln Ile Ile Tyr Ile Ser Ser
   130 135 140
   Val Ala Gly Leu His Gly Ala Ala Gln Val Ala Val Tyr Ser Ala Ser
   145 150 155 160
   Lys Ala Gly Leu Asp Gly Phe Met Arg Ser Val Ala Arg Glu Val Gly
   165 170 175
   Pro Lys Gly Ile His Val Asn Ser Ile Asn Pro Gly Tyr Thr Lys Thr
   180 185 190
   Glu Met Thr Ala Gly Ile Glu Ala Leu Pro Asp Leu Pro Ile Lys Gly
   195 200 205
   Trp Ile Glu Pro Glu Ala Ile Ala Asp Ala Val Leu Phe Leu Ala Lys
   210 215 220
   Ser Lys Asn Ile Thr Gly Thr Asn Ile Val Val Asp Asn Gly Leu Ile
   225 230 235 240
   Ala
<210> 3
   <211> 723
   <212> DNA
   <213> Candida magnoliae
<220>
   <221> CDS
   <222> (1) .. (723)
   <223>
<400> 3
   atg aca tct aca cct aat gcc ctt gtc acg gga ggc agc cgc ggc att       48
   Met Thr Ser Thr Pro Asn Ala Leu Val Thr Gly Gly Ser Arg Gly Ile
   1               5                   10                  15
   ggc gct tcc gcc gcc atc aag ctg gct caa gaa ggg tac agc gtc acg       96
   Gly Ala Ser Ala Ala Ile Lys Leu Ala Gln Glu Gly Tyr Ser Val Thr
               20                  25                  30
   ctg gcg tcc cgc gac ctt gag aaa ctt aac gag gtc aag gac aag ctg      144
   Leu Ala Ser Arg Asp Leu Glu Lys Leu Asn Glu Val Lys Asp Lys Leu
           35                  40                  45
   cca atc gtg agg ggt gga cag aaa cac tac gtt tgg caa ctc gat ctt      192
   Pro Ile Val Arg Gly Gly Gln Lys His Tyr Val Trp Gln Leu Asp Leu
       50                  55                  60
   gcc gat gta ttg gct gca tcg tct ttc aag gcg gct cct ctg ccg gcc      240
   Ala Asp Val Leu Ala Ala Ser Ser Phe Lys Ala Ala Pro Leu Pro Ala
   65                  70                  75                  80
   agc agc tac gat ttg ttt gtt tcg aac gcc gga att gcc cag ttc tcg      288
   Ser Ser Tyr Asp Leu Phe Val Ser Asn Ala Gly Ile Ala Gln Phe Ser
                   85                  90                  95
   ccc acg gca gag tat act aat agt gag tgg ctg aac att atg acc att      336
   Pro Thr Ala Glu Tyr Thr Asn Ser Glu Trp Leu Asn Ile Met Thr Ile
               100                 105                 110
   aac tta gtg tcc ccg att gcc ctg acg aag gct ctt ttg cag gcc gtt      384
   Asn Leu Val Ser Pro Ile Ala Leu Thr Lys Ala Leu Leu Gln Ala Val
           115                 120                 125
   tct ggg agg tcg agc gag aac ccg ttt cag atc gta ttc atc tcg tcg      432
   Ser Gly Arg Ser Ser Glu Asn Pro Phe Gln Ile Val Phe Ile Ser Ser
       130                 135                 140
   gtt gca gca cta cgt ggc gtt gca caa acg gcc gtc tac agt gcg tcg      480
   Val Ala Ala Leu Arg Gly Val Ala Gln Thr Ala Val Tyr Ser Ala Ser
   145                 150                 155                 160
   aag gct ggt act gat gga ttc gca cgc tca ctt gct cgc gaa cta ggt      528
   Lys Ala Gly Thr Asp Gly Phe Ala Arg Ser Leu Ala Arg Glu Leu Gly
                   165                 170                 175
   cct caa ggc gtc cat gtg aac gtg gtg aac cct ggc tgg act aag aca      576
   Pro Gln Gly Val His Val Asn Val Val Asn Pro Gly Trp Thr Lys Thr
               180                 185                 190
   gac atg acg gaa gga gtc gaa acc cca aag gac atg ccc att aag ggc      624
   Asp Met Thr Glu Gly Val Glu Thr Pro Lys Asp Met Pro Ile Lys Gly
           195                 200                 205
   tgg atc cag cct gag gca att gct gat gct gta gta ttc ctt gcg agg     672
   Trp Ile Gln Pro Glu Ala Ile Ala Asp Ala Val Val Phe Leu Ala Arg
       210                 215                 220
   tcg aaa aac att acc ggc gcg aat att gta gtg gac aat ggt ttc tcg     720
   Ser Lys Asn Ile Thr Gly Ala Asn Ile Val Val Asp Asn Gly Phe Ser
   225                 230                 235                 240
   acg                                                                 723
   Thr
<210> 4
   <211> 241
   <212> PRT
   <213> Candida magnoliae
<400> 4
   Met Thr Ser Thr Pro Asn Ala Leu Val Thr Gly Gly Ser Arg Gly Ile
   1               5                   10                  15
   Gly Ala Ser Ala Ala Ile Lys Leu Ala Gln Glu Gly Tyr Ser Val Thr
               20                  25                  30
   Leu Ala Ser Arg Asp Leu Glu Lys Leu Asn Glu Val Lys Asp Lys Leu
           35                  40                  45
   Pro Ile Val Arg Gly Gly Gln Lys His Tyr Val Trp Gln Leu Asp Leu
       50                  55                  60
   Ala Asp Val Leu Ala Ala Ser Ser Phe Lys Ala Ala Pro Leu Pro Ala
   65                  70                  75                  80
   Ser Ser Tyr Asp Leu Phe Val Ser Asn Ala Gly Ile Ala Gln Phe Ser
                   85                  90                  95
   Pro Thr Ala Glu Tyr Thr Asn Ser Glu Trp Leu Asn Ile Met Thr Ile
               100                 105                 110
   Asn Leu Val Ser Pro Ile Ala Leu Thr Lys Ala Leu Leu Gln Ala Val
           115                 120                 125
   Ser Gly Arg Ser Ser Glu Asn Pro Phe Gln Ile Val Phe Ile Ser Ser
       130                 135                 140
   Val Ala Ala Leu Arg Gly Val Ala Gln Thr Ala Val Tyr Ser Ala Ser
   145                 150                 155                 160
   Lys Ala Gly Thr Asp Gly Phe Ala Arg Ser Leu Ala Arg Glu Leu Gly
                   165                 170                 175
   Pro Gln Gly Val His Val Asn Val Val Asn Pro Gly Trp Thr Lys Thr
               180                 185                 190
   Asp Met Thr Glu Gly Val Glu Thr Pro Lys Asp Met Pro Ile Lys Gly
           195                 200                 205
   Trp Ile Gln Pro Glu Ala Ile Ala Asp Ala Val Val Phe Leu Ala Arg
       210                 215                 220
   Ser Lys Asn Ile Thr Gly Ala Asn Ile Val Val Asp Asn Gly Phe Ser
   225                 230                 235                 240
   Thr